# EUROPÄISCHE PATENTANMELDUNG

(11) **EP 1 000 585 A2**
(43) Veröffentlichungstag der Anmeldung: **17.05.2000**
(21) Anmeldenummer: 99122452.8
(22) Anmeldetag: 11.11.1999
(51) Int. Cl.: A61B 17/60

(54) **System zum Fixieren von Knochenfrakturen**

(30) Priorität: 16.11.1998 DE 29820434 U
(71) Anmelder: Dunsch-Herzberg, Renate, D-22880 Wedel (DE); Voss, Gudrun, D-25491 Hetlingen (DE)
(72) Erfinder: Herzberg, Wolfgang, Dr., D-22880 Wedel (DE)
(74) Vertreter: Richter, Werdermann & Gerbaulet

(57) **Zusammenfassung**

Das System (100) zum Fixieren von Knochenfrakturen (10), insbesondere instabiler distaler Radiusfrakturen, in einer vorbestimmten Repositionsstellung weist ein flexibles schlauchförmiges Teil (14) aus Kunststoff, das außerhalb eines Patienten gegenüber zu fixierenden Knochenfragmenten anordbar ist, in den verschiedenen Knochenfragmenten befestigte Drähte (16), Nägel und/oder Schrauben, die mit dem flexiblen schlauchförmigen Teil (14) verbindbar sind, und einen aushärtbaren Werkstoff auf, der in das flexible schlauchförmige Teil (14) derart füllbar ist, dass nach dem Aushärten des Werkstoffes mit den Drähten (16), Nägeln und/oder Schrauben ein festes Gestell gebildet ist, das die Knochenfragmente zum Zusammenwachsen in der vorbestimmten Repositionsstellung zusammenhält, wobei an einem ersten Endes (20) des flexiblen schlauchförmigen Teiles (14) eine Verschlussklemme (22) vorgesehen ist, die das erste Ende (20) verschließt und ein Aufnahmeteil (24) für einen Abschnitt des flexiblen schlauchförmigen Teiles (14) aufweist, das derart ausgebildet ist, dass es das flexible schlauchförmige Teil (14) in einem Abschnitt nahe eines zweiten Endes (26) desselben fixierend aufnimmt, wobei das zweite Ende offen (26) zugänglich bleibt.

## Beschreibung

Die Erfindung betrifft eine System zum Fixieren von Knochenfrakturen, insbesondere instabiler distaler Radiusfrakturen, in einer vorbestimmten Repositionsstellung welches folgendes aufweist, mindestens ein flexibles schlauchförmiges Teil aus Kunststoff, welches außerhalb eines Patienten gegenüber zu fixierenden Knochenfragmenten anordbar ist, in den verschiedenen Knochenfragmenten befestigte Drähte, Nägel und/oder Schrauben, welche mit dem flexiblen schlauchförmigen Teil verbindbar sind, und einen aushärtbaren Werkstoff, welcher in das flexible schlauchförmige Teil derart füllbar ist, dass nach dem Aushärten des Werkstoffes mit den Drähten, Nägeln und/oder Schrauben ein festes Gestell gebildet ist, welches die Knochenfragmente zum Zusammenwachsen in der vorbestimmten Repositionsstellung zusammenhält, gemäß dem Oberbegriff des Anspruchs 1.

Aus der EP 0 260 484 B1 ist ein gattungsgemäßer "Fixateur externe" bekannt, bei dem die beiden freien Enden des schlauchförmigen Teiles über ein Y-förmiges oder T-förmiges Verbindungsstück miteinander verbunden sind.

Es ist Aufgabe der vorliegenden Erfindung, ein System der obengenannten Art dahingehend zu verbessern, dass beim Befüllen des schlauchförmigem Teiles eine einfache Kontrollmöglichkeit bzgl. des Fortschritts der Füllung des flexiblen schlauchförmigen Teiles zu Verfügung steht und ein Einschluss von größeren Luftblasen im mittleren Bereich des schlauchförmigen Teiles weit entfernt von den Enden bei der Befüllung des flexiblen schlauchförmigen Teiles wirksam und ohne besondere Vorkehrungen beim Befüllen vermieden ist, so das über den gesamten Bereich des flexiblen schlauchförmigen Teiles eine ausreichende Stabilität gewährleistet ist.

Diese Aufgabe wird durch ein System der o.g. Art mit den in Anspruch 1 gekennzeichneten Merkmalen gelöst.

Dazu ist es erfindungsgemäß vorgesehen, dass an einem ersten Endes des flexiblen schlauchförmigen Teiles eine Verschlussklemme vorgesehen ist, welche das erste Ende verschließt und ein Aufnahmeteil für einen Abschnitt des flexiblen schlauchförmigen Teiles aufweist, welches derart ausgebildet ist, dass es das flexible schlauchförmige Teil in einem Abschnitt nahe eines zweiten Endes desselben fixierend aufnimmt, wobei das zweite Ende offen zugänglich bleibt.

Dies hat den Vorteil, dass nach einer vereinfachten Montage des erfindungsgemäßen Systems über das zweite offene Ende des flexiblen schlauchförmigen Teiles eine einfache Befüllung desselben mit dem aushärtbaren Werkstoff gewährleistet ist. Ferner wird durch die freie Wahlmöglichkeit des Abschnittes benachbart zum zweiten Ende, welcher in der Verschlussklemme fixiert wird, eine größere Flexibilität und Anpassbarkeit an unterschiedlichste Repositionsaufgaben sowie eine universellere Einsetzbarkeit des erfindungsgemäßen Systems erzielt. Ferner ergibt sich der Vorteil, dass durch entsprechend langes Überstehen des zweiten offenen Endes über die Verschlussklemme hinaus ein Zuführort für den aushärtbaren Werkstoff weit weg von der steril zu haltenden Operationsstelle lokalisiert ist, so dass entsprechende Zuführmittel für den aushärtbaren Werkstoff selbst nicht steril sein müssen und von einem unsterilen Helfer betätigt bzw. zugeführt werden können. Durch das Einbringen des aushärtbaren Werkstoffes von nur dem einen offenen Ende aus wird der Werkstoff durch das gesamten flexible schlauchförmige Teil gepresst und schiebt ggf. vorhandenen Luftblasen vor sich her, so dass diese allenfalls im Bereich des ersten Endes vorhanden sind. Zweckmäßigerweise ist zusätzlich eine Entlüftungseinrichtung am ersten Ende vorgesehen, so dass derartige Luftblasen aus dem flexiblen schlauchförmigen Teil entweichen können.

Vorzugsweise Weitergestaltungen der Vorrichtung sind in den Ansprüchen 2 bis 14 beschrieben.

Zum Kontrollieren eines Fortschrittes der Befüllung des flexiblen schlauchförmigen Teiles mit einem aushärtbaren Werkstoff weist die Verschlussklemme ein Sichtfenster auf, welches derart angeordnet ist, dass es einen visuellen Einblick in das erste verschlossene Ende des flexiblen schlauchförmigen Teiles gewährleistet.

In einer bevorzugten Ausführungsform ist die Verschlussklemme als einstückiges Kunststoffspritzgussteil ausgebildet.

Zeckmäßigerweise sind zum Verbinden der Drähte, Nägel und/oder Schrauben mit dem flexiblen schlauchförmigen Teil eine entsprechende Anzahl von Schlauchverbindungsklemmen vorgesehen.

Eine beliebige angeordnete Verbindung des flexiblen schlauchförmigen Teiles mit den Drähten, Nägeln und/oder Schrauben erzielt man dadurch, dass die Schlauchverbindungsklemmen zwei mit einem Scharnier, beispielsweise einem Filmscharnier, verbundene Elemente umfassen, wobei jedes Element einen Teil einer Aufnahme für das flexible schlauchförmige Teil und eine Aufnahme für die Drähte, Schrauben und/oder Nägel aufweist, wobei an den Elementen ein Verriegelungsmechanismus angeordnet ist, welcher die beiden Elemente beim Zusammenschwenken um das Scharnier miteinander derart verriegelnd verbindet, dass der flexibel schlauchförmige Teil fest mit der Schlauchverbindungsklemme verbunden ist. Hierbei ist es besonders von Vorteil, dass beliebige, frei wählbare Abschnitte des flexiblen schlauchförmigen Teiles mit den jeweiligen Drähten, Nägeln und/oder Schrauben verbindbar sind.

Zum Ausbilden eines festen Gestelles weisen die Schlauchverbindungsklemmen ferner eine verpressbare Vorrichtung auf, welche mit einem Werkzeug verpresst eine unlösbar Verbindung der beiden Elemente der Schlauchverbindungsklemmen mit dem Draht, Nagel oder der Schraube ausbildet.

In einer bevorzugten Ausführungsform weist die verpressbare Vorrichtung je einen Klemmkegel für ein Element der Schlauchverbindungsklemme auf, wobei jeder Klemmkegel in einer Durchgangsöffnung des entsprechenden Elementes für einen jeweiligen Draht, Nagel oder eine jeweilige Schraube abgeordnet ist und diese Durchgangsöffnung Eingriffsmittel aufweist, welche bei Einpressen des Klemmkegels in die Durchgangsöffnung mit entsprechenden Eingriffsmitteln am Klemmkegel zusammenwirkend eine feste und unlösbare Verbindung der Elemente mit dem jeweiligen Draht, Nagel bzw. der jeweiligen Schraube ausbilden.

In einer bevorzugten Ausführungsform sind die Schlauchverbindungsklemmen als einstückiges Kunststoffspritzgussteil ausgebildet. Zweckmäßigerweise ist das Scharnier als Filmscharnier ausgebildet.

Der aushärtbarer Werkstoff ist ein Zweikomponentenwerkstoff. Zum Zuführen des aushärtbaren Werkstoffes ist in besonders vorteilhafter Weise eine insbesondere handbetätigbare Kartuschenpumpe vorgesehen, welche eine Kartusche mit Austragsöffnung für den aushärtbaren Werkstoff aufweist. Zur Abschirmung der unsterilen Pumpe vom steril Operationsfeld ist eine sterile Schutzkappe vorgesehen, welche über die Kartusche schiebbar ist.

Zum Austragen von Zweikomponentenwerkstoffen, welche nach Vermischung aushärten weist die Kartusche zwei Kammern für die zwei Komponenten des aushärtbaren Werkstoffes auf, wobei die Austragsöffnung der Kartusche mit beiden Kammern in Verbindung steht und eine Mischspitze vorgesehen ist, welche mittels eines Befestigungsmechanismus, welcher insbesondere ein Bajonettverschluss ist, lösbar mit der Austragsöffnung verbindbar ist, wobei in der Mischspitze eine Vermischung der beiden Komponenten vor dem Einbringen in das flexible schlauchförmige Teil erfolgt.

Nachstehend wird die Erfindung anhand der beigefügten Zeichnungen näher erläutert. Diese zeigen in
- Fig. 1: eine bevorzugte Ausführungsform eines erfindungsgemäßen Systems in perspektivischer Ansicht,
- Fig. 2: eine Verschlussklemme in perspektivischer Ansicht,
- Fig. 3: in einer weiteren perspektivischen Ansicht,
- Fig. 4 bis 6: eine Schlauchverbindungsklemme in verschiedenen perspektivischen Ansichten,
- Fig. 7: die Schlauchverbindungsklemme von Fig. 6 in einer Schnittansicht,
- Fig. 8: einen Klemmkegel für eine Schlauchverbindungsklemme gemäß Fig. 6 und 7 in perspektivischer Ansicht,
- Fig. 9: eine sterile Schutzkappe in perspektivischer Ansicht,
- Fig. 10: einen manuell betätigbaren Pumpmechanismus für eine Kartuschenpumpe in Seitenansicht und
- Fig. 11A bis 11C: mit aufeinanderfolgenden Handhabungsschritten A bis M des erfindungsgemäßen Systems.

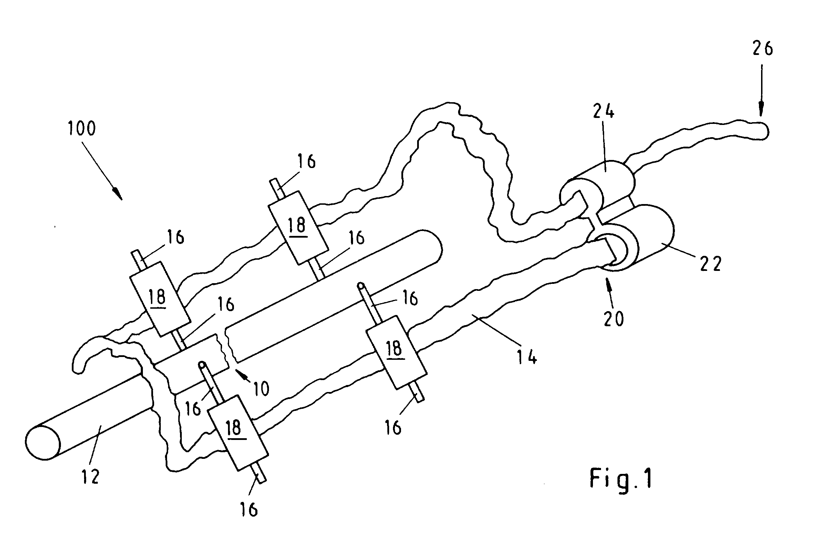

Die in Fig. 1 dargestellte bevorzugte Ausführungsform eines erfindungsgemäßen Systems 100 zum Fixieren einer Knochenfraktur 10 eines beispielsweise menschlichen Knochens 12 während eines Heilungsprozesses umfasst ein flexibles schlauchförmiges Teil 14 und Drähte 16, welche sowohl mit dem Knochen 12 bzw. dessen Fragmenten als auch mit dem Schlauch 14 verbunden sind. Zur Verbindung der Drähte 16 mit dem Schlauch 14 sind nachfolgend näher beschriebene Schlauchverbindungsklemmen 18 vorgesehen. An einem ersten Ende 20 des Schlauches 14 ist eine dieses erste Ende 20 verschließende, nachfolgend näher beschriebene Verschlussklemme 22 angeordnet. An dieser Verschlussklemme 22 ist ein Aufnahmeteil 24 für einen Abschnitt des Schlauches 14 benachbart zu einem zweiten Ende 26 angeordnet, wobei das zweite Ende 26 des Schlauches 14 offen bleibt. Der Schlauch 14 bildet zusammen mit den Drähten 16 nach dem Aushärten eines in den Schlauch 14 eingebrachten Werkstoffes ein festes Gestell, welches die Knochenfragmente des Knochens 12 während eines Heilungsprozesses bzw. während des wieder Zusammenwachsens der Knochenfragmente fixiert.

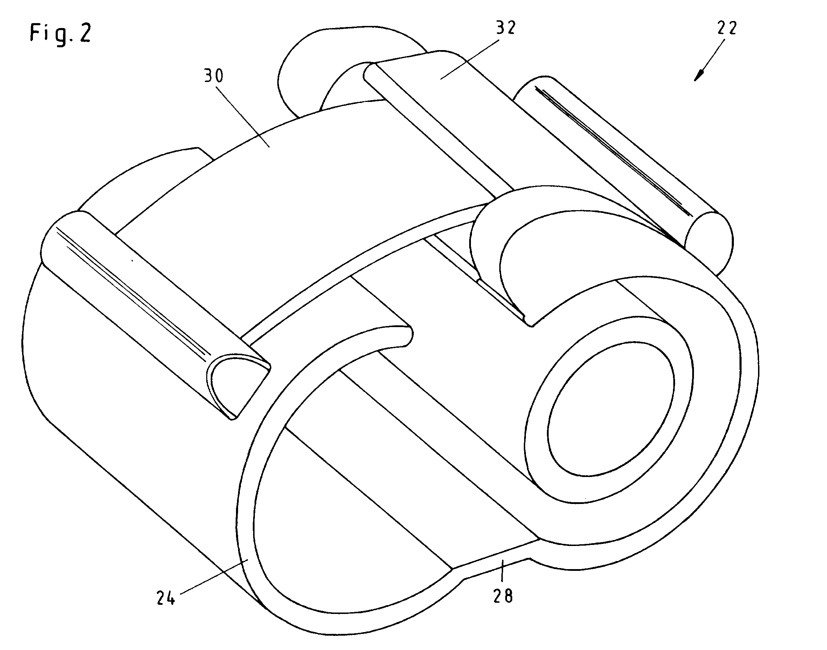

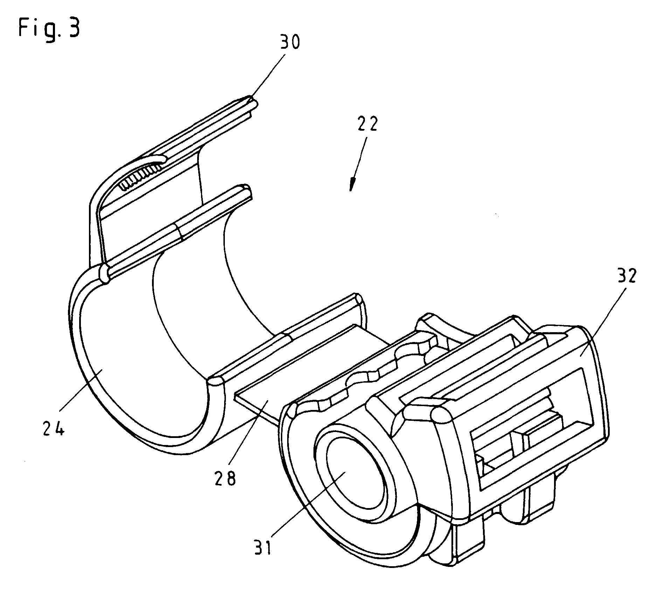

Die in den Fig. 2 und 3 dargestellte bevorzugte Ausführungsform einer Verschlussklemme 22 umfasst ein Filmscharnier 28, eine am Aufnahmeteil 24 angeordnete Verriegelungslasche 30 und ein Verriegelungselement 32 zur Aufnahme der Verriegelungslasche 30. Das Aufnahmeteil 24 ist um das Filmscharnier 28 schwenkbar, so dass die Verriegelungslasche 30 in das Verriegelungselement greifend eine unlösbare Verbindung ausbildet. Im Bereich des verschlossenen Endes 20 weist die Verschlussklemme 22 ein Sichtfenster 31 auf, durch welches in das Ende 20 des Schlauches 14 hinein gesehen werden kann. Auf diese Weise ist erkennbar, wann der in das offenen Ende 26 eingepresste aushärtbare Werkstoff den Schlauch 14 vollständig ausfüllt und am geschlossenen Ende 20 ankommt.

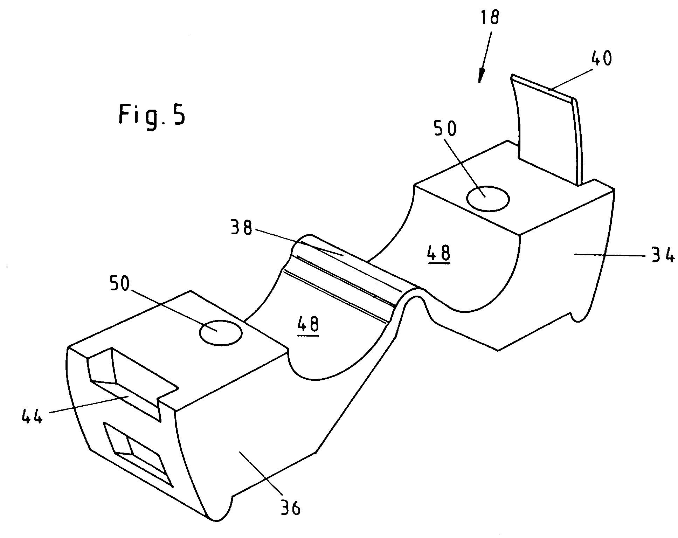

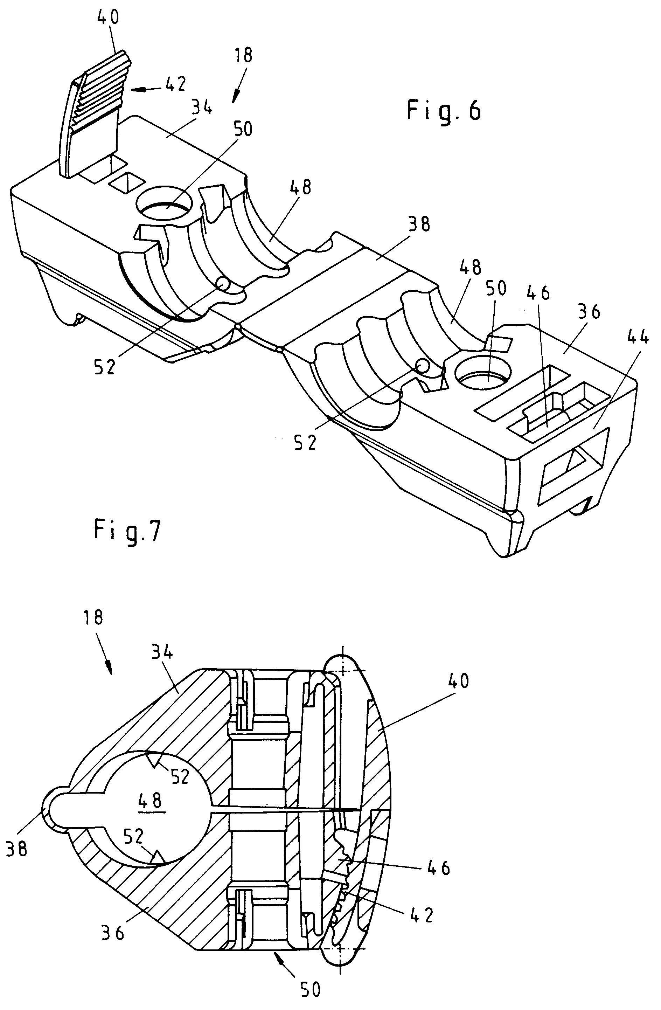

Die in Fig. 3 bis 7 dargestellte Schlauchverbindungsklemme 18 ist aus zwei Elementen 34 und 36 gebildet, welche über ein Filmscharnier 38 miteinander verbunden sind. Am ersten Element 34 eine Verriegelungslasche 40 mit Rastzähnen 42 ausgebildet, während am zweiten Element 36 ein Verriegelungselement 44 zur Aufnahme der Verriegelungslasche 40 mit einer entsprechenden Rastnase 46 ausgebildet ist, welche zusammenwirkend mit den Rastzähnen eine feste ggf. unlösbare Verbindung ausbildet. In jedem Element 34, 36 ist eine Hälfte einer Schlauchaufnahme 48 ausgebildet. Ferner umfasst jedes Element 34, 36 eine Durchgangsöffnung 50 zum hindurch stecken eines jeweiligen Drahtes, mit dem die jeweilige Schlauchverbindungsklemme 18 zu verbinden ist.

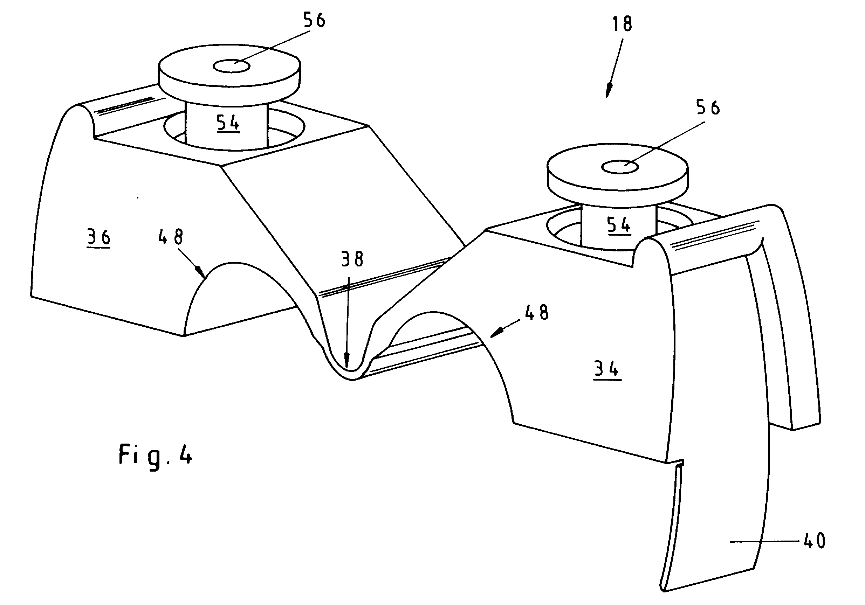

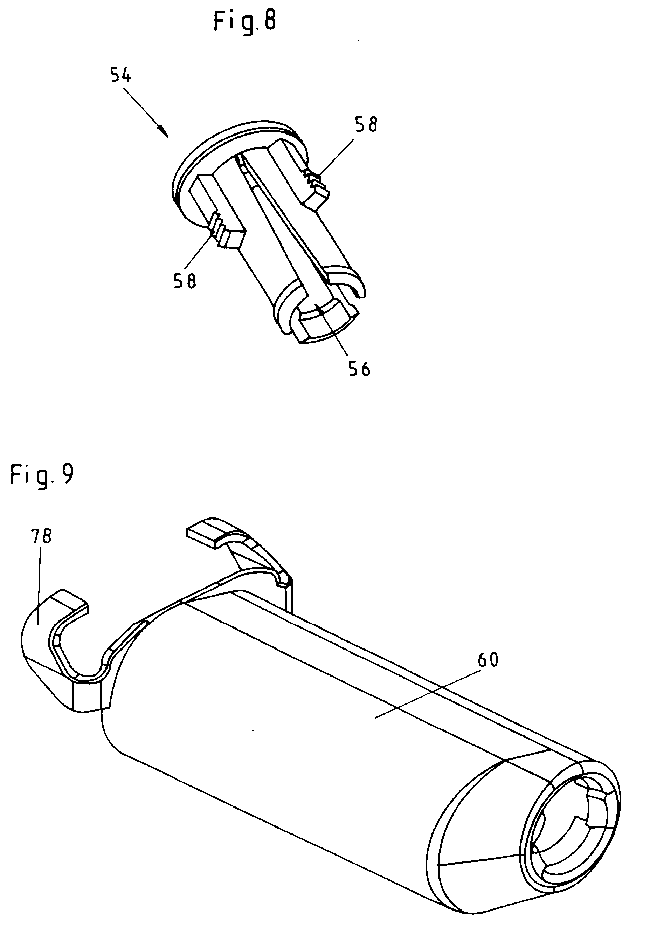

Durch die feste Verbindung der Verriegelungslasche 40 mit dem Verriegelungselement 44 ist eine feste Halterung des Schlauches 14 in den zusammen geschwenkten Elementen gewährleistet. Entsprechend in der Schlauchaufnahme 48 ausgebildete Erhebungen 52 verhindern ein axiales Verrutschen des Schlauches 14 in der Schlauchaufnahme 48. In die Durchgangsöffnungen 50 sind Klemmkegel 54 eingesteckt, wie in Fig. 4 dargestellt. Diese Klemmkegel 54 weisen eine mittige Durchgangsöffnung 56 zum hindurch stecken eines jeweiligen Drahtes 16 auf. Durch Einpressen der Klemmkegel 54 in die Durchgangsöffnungen 56 wird eine feste Verbindung zwischen dem hindurch gesteckten Draht 16 und der Schlauchverbindungsklemme 18 hergestellt. Hierzu umfasst der Klemmkegel 54, wie in Fig. 8 dargestellt, entsprechende Eingriffsmittel 58, welche bei Einpressen des Klemmkegels 54 in die Durchgangsöffnung 50 mit entsprechenden Eingriffsmitteln in der Durchgangsöffnung 50 zusammenwirkend eine feste und unlösbare Verbindung der Elemente 34, 36 mit dem jeweiligen Draht 16 ausbilden.

Fig. 9 zeigt eine sterile Schutzabdeckung 60 für eine Kartusche einer später näher beschriebenen Kartuschenpumpe zum Einbringen des aushärtbaren Werkstoffes in das zweite offene Ende 26 des Schlauches 14. Da dieses zweite offene Ende 26 vom steril zu haltenden Operationsbereich weit entfernt ist, muss die Kartuschenpumpe selbst nicht steril gehalten sein. Es genügt statt dessen das Vorsehen der sterilen Schutzabdeckung 60 über einem dem Operationsfeld zugewandten Teil der Pumpe, welches die von der Pumpe zu leerende Kartusche ist. Zusätzlich ist lediglich eine an einer Austragsöffnung der Kartusche befestigte und aus der sterilen Schutzabdeckung herausragende und in das offenen zweite Ende 26 einzuführende Mischspitze steril zu halten.

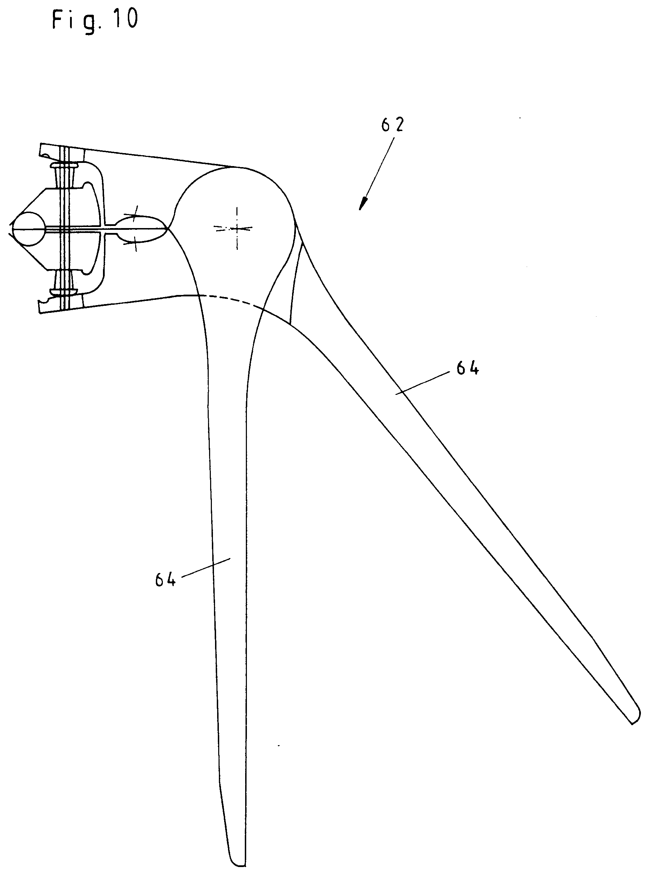

Fig. 10 zeigt beispielhaft einen manuellen Pumpmechanismus 62 einer Kartuschenpumpe. Dieser Pumpmechanismus 62 umfasst zwei manuell zusammendrückbare Hebelarme 64, wobei wenigstens einer als Handgriff zum Halten der Kartuschenpumpe dient. An einem Kopfbereich 66 ist eine in Fig. 10 nicht dargestellte Kartusche anordbar, aus der durch abwechselndes Zusammendrücken und wieder loslassen der Hebelarme 64 ein aushärtbares Material ausgetragen wird.

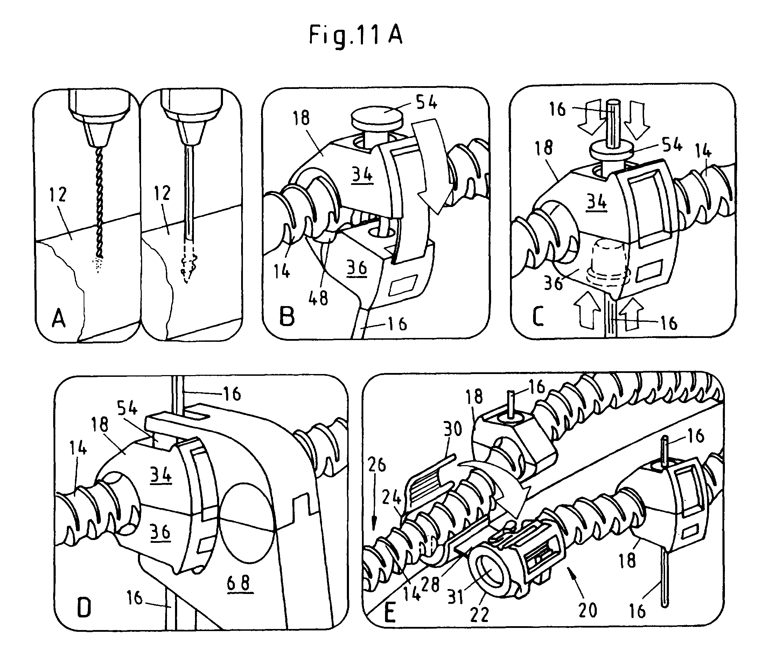

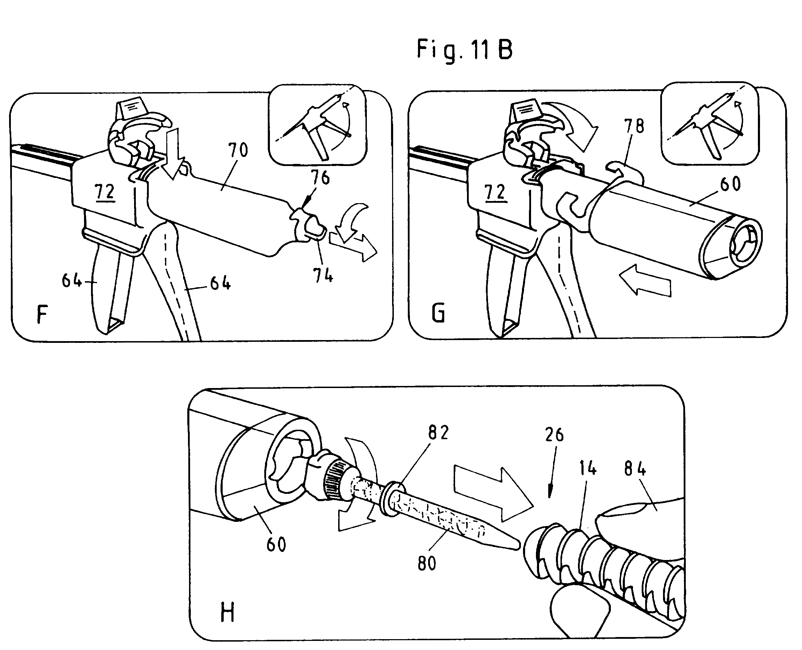

Weitere Merkmale und Vorteile des erfindungsgemäßen Systems ergeben sich aus der nachfolgenden Beschreibung der Handhabung anhand der Fig. 11A bis 11M.

Das erfindungsgemäße System dient speziell zur Behandlung instabiler distaler Radiusfrakturen. Der Schlauch 14 ist beispielsweise ein Glasfaser-Kunststoff-Verbund mit stahlänlichen mechanischen Eigenschaften, aber trotzdem Röntgentransparent. Bei beispielsweise nur 70g Eigengewicht ergibt sich ein hoher Tragekomfort. Gleichzeitig ist eine einfache Montage in räumlich frei wählbaren Ebenen möglich. Bei zusätzlicher Verwendung eines Kettenfixateurs sind beliebig reproduzierbare Fraktur-Repositionen möglich. Es gibt keine Verschleißteile und keine Rückführungslogistik.

Nach einer Anästhesie (Plexus-, i.v.-Regionalanästhesie oder Vollnarkose) wird der Patient mit leicht erhöhtem Oberkörper in Rückenlage mit ausgelagertem Arm positioniert. Der Arm wird in der Ellenbeuge mit einer Rolle gelagert und die Finger 1, 2 und 3 über Mädchenfänger mit insgesamt 3 kg Zug extendiert. Vorteilhaft ist eine kopfwärtige Bildwandlerposition. Der Monitor steht bevorzugt auf der Gegenseite. Ein Assistent ist hierbei nicht erforderlich. Die durch Extension weitestgehende Reposition ist nun in nachfolgend beschriebener Weise mittels des erfindungsgemäßen Systems fixierbar.

An geeigneten Stellen des Knochens 12 werden proximal zur Fraktur an der dorsalen Radiusseite senkrecht zur Schaftachse Pins bzw. Drähte 16 gesetzt (Fig. 11A). Nach dem Bohren mit beispielsweise 1,5 mm durch eine Bohrführungshülse wird der Pin 16 manuell eingedreht und über eine Distanz-Bohrführung ein zweiter Pin 16 proximal davon gesetzt. Unter Röntgenkontrolle wird die Lage des Pins 16 kontrolliert.

Nach dem Setzen aller weiteren Pins 16 werden die offenen Schlauchverbindungsklemmen 18 mit dem Element 36 auf die Pins 16 gesetzt (Fig. 11B). Der Spiralschlauch 14 wird in die Aufnahmen 48 der offenen Schlauchverbindungsklemmen 18 gelegt, wobei das erste Ende 20 mit der Verschlussklemme 22 ca. 4 cm vom proximalen Pin 16 angeordnet wird. Der Schlauch 14 hat zweckmäßigerweise eine Längenreserve, um die Reposition zu ermöglichen. Die Schlauchverbindungsklemmen 18 werden geschlossen.

Bei Einsatz eines Kettenfixateurs wird dieser entspannt von außen unter dem Glasfaser/Spiralschlauch 14 auf die Pins 16 montiert. Nach Erreichen einer gewünschten Frakturreposition wird der Kettenfixateur gespannt. Dies erzielt eine zeitweilige rigide Position.

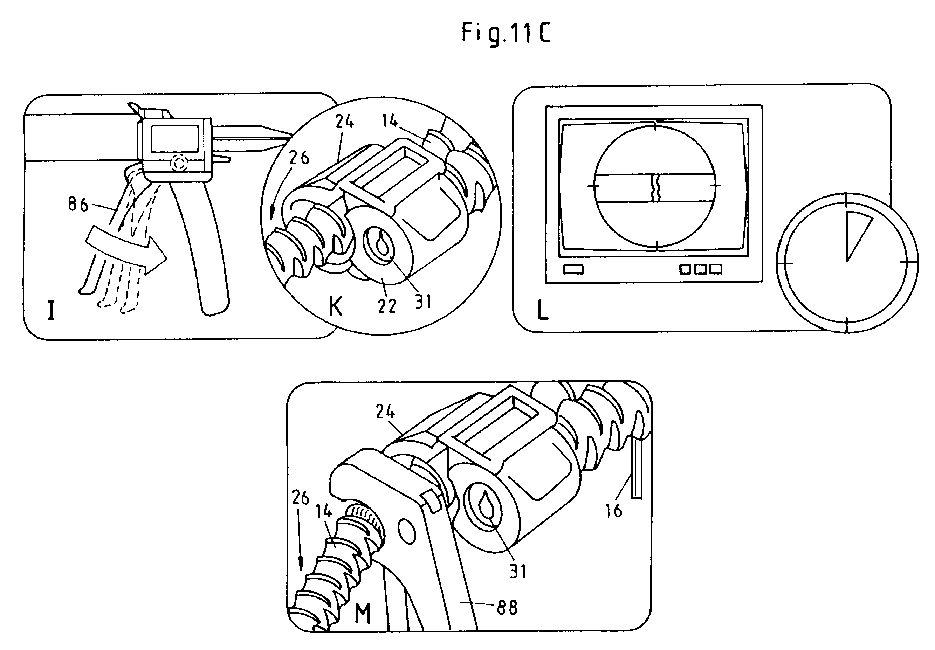

Mittels eines Werkzeuges 68 werden die jeweiligen Klemmkonen 54 eingedrückt und verbinden den jeweiligen Pin 16 mit der Schlauchverbindungsklemme 18 (Fig. 11C und 11D). Der Schlauch 14 wird dann je nach erforderlicher Schlauchlänge mit einem Abschnitt benachbart zum zweiten offenen Ende 26 in die Verschlussklemme 22 gelegt und diese geschlossen (Fig. 11E).

Ein unsteriler OP-Helfer setzt, wie in Fig. 11F dargestellt, vorsichtig eine unsterile Harz-Kartusche 70 in die Kartuschenpumpe 72 und entfernt einen Verschluss 74 an der Austragsöffnung 76 der Kartusche 70. Dabei ist die Kartuschenpumpe 74 schräg nach oben zu halten.

Danach setzt der sterile instrumentiernende Operateur, wie in Fig. 11G dargestellt, die sterile Kartuschenschutzkappe 60 auf und verriegelt diese mit einem Überwurf 78 an der Kartuschenpumpe 72.

Eine sterile Mischspitze 80 wird, wie in Fig. 11H dargestellt, in die Harz-Kartusche 70 eingedreht und danach vollständig in das zweite offenen Ende 26 des Schlauches 14 eingeschoben. Eine Gummidichtung 82 verhindert ein Zurücklaufen des Harzgemisches beim Pumpen. Eine freie Hand 84 des Operateurs sichert die Verbindung von Mischspitze 80 und Spiralschlauch 14.

Durch ruhige Pumpbewegungen, wie in Fig. 11l und 11k angedeutet, wird das Gemisch in den Schlauch 14 gepresst. Nach dem Durchziehen des Pumpengriffes 86 wird dieser kurz in angezogener Stellung gehalten, um einen gleichmäßigen Druckausgleich zu gewährleisten. Erreicht der aushärtbare Werkstoff in Form eines Harzgemisch aus zwei Komponenten das Sichtfenster 31 am Schlauchende 20 wird der Pumpvorgang abgebrochen und der Pumpenhandgriff 86 mit Kartusche 70 abgelegt oder mittels Tuchklemme fixiert. Die Harz-Kartusche 70 ist in diesem Stadium nicht vom Schlauch 14 zu lösen. Bei einer Aushärtzeit von beispielsweise 5 bis 10 Minuten, wie in Fig. 11L angedeutet, verbleiben nach einer Pumpzeit von 1 bis 2 Minuten noch 4 bis 8 Minuten zur Aushärtung und Reposition. Die Aushärtzeit ist dabei Temperaturabhängig. Das Harzgemisch härtet unter Erwärmung aus und erreicht in diesen ersten 5 bis 10 Minuten ca. 90% seiner Festigkeit. Innerhalb der nächsten 12 Stunden erfolgt eine vollständige Aushärtung.

Das überstehende, zum zweiten offenen Ende 26 benachbarte Schlauchende wird nun mit einem Schlauchschneider 88 abgetrennt. Ein Stössel der Kartuschenpumpe 72 wird zurück gezogen, die leere Harz-Kartusche 70 entnommen und ohne besondere Vorkehrungen mit dem Klinikmüll entsorgt.

### BEZUGSZEICHENLISTE

- 100: System
- 10: Knochenfraktur
- 12: Knochen
- 14: flexibles schlauchförmiges Teil
- 16: Drähte
- 18: Schlauchverbindungsklemmen
- 20: erstes Ende
- 22: Verschlussklemme
- 24: Aufnahmeteil für einen Abschnitt des Schlauches
- 26: zweites Ende
- 28: Filmscharnier
- 30: Verriegelungslasche
- 31: Sichtfenster
- 32: Verriegelungselement
- 34: erstes Element
- 36: zweites Element
- 38: Filmscharnier
- 40: Verriegelungslasche
- 42: Rastzähne
- 44: Verriegelungselement
- 46: Rastnase
- 48: Schlauchaufnahme
- 50: Durchgangsöffnung
- 52: Erhebungen
- 54: Klemmkegel
- 56: Durchgangsöffnung der Klemmkegel
- 58: Eingriffsmittel
- 60: sterile Schutzabdeckung
- 62: Pumpmechanismus
- 64: Hebelarme
- 66: Kopfbereich
- 68: Werkzeug
- 70: unsterile Harz-Kartusche
- 72: Kartuschenpumpe
- 74: Verschluss
- 76: Austragsöffnung
- 78: Überwurf
- 80: Mischspitze
- 82: Gummidichtung
- 84: freie Hand
- 86: Pumpengriff
- 88: Schlauchschneider

## Patentansprüche

1. System (100) zum Fixieren von Knochenfrakturen (10), insbesondere instabiler distaler Radiusfrakturen, in einer vorbestimmten Repositionsstellung welches folgendes aufweist, mindestens ein flexibles schlauchförmiges Teil (14) aus Kunststoff, welches außerhalb eines Patienten gegenüber zu fixierenden Knochenfragmenten anordbar ist, in den verschiedenen Knochenfragmenten befestigte Drähte (16), Nägel und/oder Schrauben, welche mit dem flexiblen schlauchförmigen Teil (14) verbindbar sind, und einen aushärtbaren Werkstoff, welcher in das flexible schlauchförmige Teil (14) derart füllbar ist, dass nach dem Aushärten des Werkstoffes mit den Drähten (16), Nägeln und/oder Schrauben ein festes Gestell gebildet ist, welches die Knochenfragmente zum Zusammenwachsen in der vorbestimmten Repositionsstellung zusammenhält,
dadurch gekennzeichnet,
dass an einem ersten Endes (20) des flexiblen schlauchförmigen Teiles (14) eine Verschlussklemme (22) vorgesehen ist, welche das erste Ende (20) verschließt und ein Aufnahmeteil (24) für einen Abschnitt des flexiblen schlauchförmigen Teiles (14) aufweist, welches derart ausgebildet ist, dass es das flexible schlauchförmige Teil (14) in einem Abschnitt nahe eines zweiten Endes (26) desselben fixierend aufnimmt, wobei das zweite Ende offen (26) zugänglich bleibt.

2. System (100) nach Anspruch 1,
dadurch gekennzeichnet,
dass die Verschlussklemme (22) ein Sichtfenster (31) aufweist, welches derart angeordnet ist, dass es einen visuellen Einblick in das erste verschlossene Ende (20) des flexiblen schlauchförmigen Teiles (14) gewährleistet.

3. System (200) nach Anspruch 1 oder 2,
dadurch gekennzeichnet,
dass die Verschlussklemme (22) als einstückiges Kunststoffspritzgussteil ausgebildet ist.

4. System (100) nach einem der vorhergehenden Ansprüche,
dadurch gekennzeichnet,
dass zum Verbinden der Drähte (16), Nägel und/oder Schrauben mit dem flexiblen schlauchförmigen Teil (14) eine entsprechende Anzahl von Schlauchverbindungsklemmen (18) vorgesehen sind.

5. System (100) nach Anspruch 4,
dadurch gekennzeichnet,
dass die Schlauchverbindungsklemmen (18) zwei mit einem Scharnier (38) verbundene Elemente (34, 36) umfassen, wobei jedes Element (34,36) einen Teil einer Aufnahme (48) für das flexible schlauchförmige Teil (14) und eine Aufnahme (50) für die Drähte (16), Schrauben und/oder Nägel aufweist, wobei an den Elementen (34, 36) ein Verriegelungsmechanismus (40, 44) angeordnet ist, welcher die beiden Elemente (34, 36) beim Zusammenschwenken um das Scharnier (38) miteinander derart verriegelnd verbindet, dass der flexibel schlauchförmige Teil (16) fest mit der Schlauchverbindungsklemme verbunden ist.

6. System (100) nach Anspruch 5,
dadurch gekennzeichnet,
dass die Schlauchverbindungsklemmen (18) ferner eine verpressbare Vorrichtung (54) aufweisen, welche mit einem Werkzeug (68) verpresst eine unlösbar Verbindung der beiden Elemente der Schlauchverbindungsklemmen mit dem Draht (16), Nagel oder der Schraube ausbildet.

7. System (100) nach einem der Ansprüche 5 oder 6,
dadurch gekennzeichnet,
dass die verpressbare Vorrichtung je einen Klemmkegel (54) für ein Element (34, 36) der Schlauchverbindungsklemme (18) aufweist, wobei jeder Klemmkegel (54) in einer Durchgangsöffnung (50) des entsprechenden Elementes (34, 36) für einen jeweiligen Draht (16), Nagel oder eine jeweilige Schraube angeordnet ist und diese Durchgangsöffnung (50) Eingriffsmittel aufweist, welche bei Einpressen des Klemmkegels (54) in die Durchgangsöffnung (50) mit entsprechenden Eingriffsmitteln (58) am Klemmkegel (54) zusammenwirkend eine feste und unlösbare Verbindung der Elemente (34, 36) mit dem jeweiligen Draht (16), Nagel bzw. der jeweiligen Schraube ausbilden.

8. System (100) nach einem der Ansprüche 5 bis 7,
dadurch gekennzeichnet,
dass das Scharnier als Filmscharnier (38) ausgebildet ist.

9. System (100) nach einem der Ansprüche 4 bis 8,
dadurch gekennzeichnet,
dass die Schlauchverbindungsklemmen (18) als einstückiges Kunststoffspritzgussteil ausgebildet sind.

10. System (100) nach einem der vorhergehenden Ansprüche,
dadurch gekennzeichnet,
dass der aushärtbarer Werkstoff ein Zweikomponentenwerkstoff ist.

11. System (100) nach einem der vorhergehenden Ansprüche,
dadurch gekennzeichnet,
dass zum Zuführen des aushärtbaren Werkstoffes eine insbesondere handbetätigbare Kartuschenpumpe (72) vorgesehen ist, welche eine Kartusche (70) mit Austragsöffnung (76) für den aushärtbaren Werkstoff aufweist.

12. System (100) nach Anspruch 11,
dadurch gekennzeichnet,
dass eine sterile Schutzkappe (60) vorgesehen ist, welche über die Kartusche (70) schiebbar ist.

13. System (100) nach Anspruch 11 oder 12,
dadurch gekennzeichnet,
dass die Kartusche (70) zwei Kammern für zwei Komponenten eines aushärtbaren Werkstoffes aufweist, wobei die Austragsöffnung (76) der Kartusche mit beiden Kammern in Verbindung steht, und eine Mischspitze (80) vorgesehen ist, welche mittels eines Befestigungsmechanismus lösbar mit der Austragsöffnung (76) verbindbar ist, wobei in der Mischspitze (80) eine Vermischung der beiden Komponenten vor dem Einbringen in das flexible schlauchförmige Teil (14) erfolgt.

14. System (100) nach Anspruch 13,
dadurch gekennzeichnet,
dass der Befestigungsmechanismus ein Bajonettverschluss ist.
